# EUROPEAN PATENT SPECIFICATION

(11) **EP 3 155 970 B1**
(45) Date of publication and mention of the grant of the patent: **31.05.2023**
(21) Application number: 16178359.2
(22) Date of filing: 07.07.2016
(51) Int. Cl.: A61B 8/00, G01N 29/24

(54) **WIRELESS PROBE AND ULTRASONIC IMAGING APPARATUS**
DRAHTLOSE SONDE UND ULTRASCHALLABBILDUNGSVORRICHTUNG
SONDE SANS FIL ET APPAREIL D'IMAGERIE ULTRASONORE

(30) Priority: 14.10.2015 KR 20150143477
(43) Date of publication of application: 19.04.2017
(73) Proprietor: Samsung Medison Co., Ltd., Hongcheon-gun, Gangwon-do, 25108 (KR)
(72) Inventor: HAN, Gun Hee, Seoul (KR); LEE, Sun Joong, Gyeonggi-do (KR); KIM, Yuri, Gwangjin-gu (KR); JIN, Gil-Ju, Seongbuk-gu (KR)
(74) Representative: Hylarides, Paul Jacques

(56) References cited:
- JP-A- 2007 222 322
- US-A1- 2004 171 935
- US-A1- 2008 114 245
- US-A1- 2010 160 786
- US-A1- 2011 061 466
- US-A1- 2012 053 465
- US-A1- 2012 141 002
- US-A1- 2014 128 739
- US-A1- 2014 180 110
- US-A1- 2015 245 823

## Description

### BACKGROUND

### 1. Field of the Invention

Embodiments of the present disclosure relate to a wireless probe for acquiring an ultrasound image and ultrasonic imaging apparatus for providing the acquired ultrasound image.

### 2. Discussion of Related Art

An ultrasonic imaging system is one of the main imaging systems applied in various areas, and especially, the ultrasonic imaging system is widely used in medical areas due to having non-invasive and non-destructive properties. The ultrasonic imaging system acquires a diagnostic image of an internal part of an object with a probe and displays the diagnostic image on an ultrasonic imaging apparatus as a two (2D) or three dimensional(3D) image.

To increase user convenience, the ultrasonic imaging apparatus and the probe are connected wirelessly in recent years, and the wireless probe has an independent power unit.

Accordingly, studies are underway for power management of the wireless probe.

Wireless probes which are automatically switched into a low-power mode are well known in the art, see documents US 2014/180110, US 2008/114245, US 2014 /128739.

### SUMMARY OF THE INVENTION

The invention is defined in the independent claims 1 and 7.

Embodiments of the present disclosure provide a wireless probe, an ultrasonic imaging apparatus, and method for controlling the same that efficiently manages power of the wireless probe.

In accordance with one aspect of the present disclosure, a wireless probe according to claim 1 is provided.

Here, the determiner may determine the condition of the wireless communication network based on at least one of transfer rate, signal quality, signal to noise ratio (SNR), and receive signal strength indication (RSSI) of a wireless signal over the wireless communication network.

Also, the determiner may determine the condition of the wireless communication network based on a signal threshold received by a user or set in advance.

Also, the determiner may determine whether the ultrasound image corresponds to an air image using image information obtained through image processing on the ultrasound image.

Also, the determiner may determine whether to switch into a low-power mode based on whether the distance information between the wireless probe and the ultrasonic imaging apparatus is within a predetermined distance, the distance information being calculated based on receive signal strength.

In accordance with another aspect of the present disclosure, an ultrasonic imaging apparatus according to claim 7 is provided.

Here, the determiner may determine the condition of the wireless communication network based on at least one of transfer rate, signal quality, signal to noise ratio (SNR), and receive signal strength indication (RSSI) of a wireless signal over the wireless communication network.

Also, the determiner may determine the condition of the wireless communication network based on a signal threshold received by a user or set in advance.

Also, the determiner may determine whether the ultrasound image corresponds to an air image using image information obtained through image processing on the ultrasound image.

Also, the determiner may determine whether to switch into a low-power mode based on whether the distance information between the wireless probe and the ultrasonic imaging apparatus is within a predetermined distance, the distance information being calculated based on receive signal strength.

In accordance with still another aspect of the present disclosure - not falling within the scope of the invention, a method for controlling a wireless probe includes: determining whether to switch into a low-power mode based on at least one of a condition of a wireless communication network, whether an ultrasound image is about an object, position information between an ultrasonic imaging apparatus and the wireless probe, and whether a predetermined waiting time has passed; and controlling switching into the low-power mode based on the determination result.

Here, the determining whether to switch into a low-power mode may include determining the condition of the wireless communication network based on at least one of transfer rate, signal quality, signal to noise ratio (SNR), and receive signal strength indication (RSSI) of a wireless signal over the wireless communication network.

Also, the determining whether to switch into a low-power mode may include determining the condition of the wireless communication network based on a signal threshold received by a user or set in advance.

Also, the determining whether to switch into a low-power mode may include determining whether the ultrasound image corresponds to an air image using image information obtained through image processing on the ultrasound image.

Also, the determining whether to switch into a low-power mode may include determining whether to switch into a low-power mode based on at least one of distance information and directional information between the wireless probe and the ultrasonic imaging apparatus.

Also, the determining whether to switch into a low-power mode may include determining whether to switch into a low-power mode based on whether the distance information between the wireless probe and the ultrasonic imaging apparatus is within a predetermined distance, the distance information being calculated based on receive signal strength.

Also, the determining whether to switch into a low-power mode may include determining whether to switch into a low-power mode based on the distance information and directional information between the wireless probe and the ultrasonic imaging apparatus calculated using triangulation or radiation pattern information.

In accordance with still another aspect of the present disclosure - not falling within the scope of the invention, a method for controlling an ultrasonic imaging apparatus includes: determining whether to switch a wireless probe into a low-power mode based on at least one of a condition of a wireless communication network that supports wireless communication with the wireless probe, whether an ultrasound image contains an object, position information between the ultrasonic imaging apparatus and the wireless probe, and whether waiting time of the wireless probe has passed; and controlling a communication unit to transmit a command signal to the wireless probe to be switched into the low-power mode, once it is determined to switch the wireless probe into the low-power mode.

### BRIEF DESCRIPTION OF THE DRAWINGS

The above and other objects, features and advantages of the present disclosure will become more apparent to those of ordinary skill in the art by describing in detail exemplary embodiments thereof with reference to the accompanying drawings, in which:
FIGS. 1 and 2 are perspective views of ultrasonic imaging systems each having a wireless probe and an ultrasonic imaging apparatus, according to different embodiments of the present disclosure.
FIG. 3 illustrates an exterior view of a wireless probe including one dimensional (1D) array transducers, according to an embodiment of the present disclosure.
FIG. 4 illustrates an exterior view of a wireless probe including two dimensional (2D) array transducers, according to an embodiment of the present disclosure.
FIG. 5 illustrates relations between a wireless probe and an ultrasonic imaging apparatus, according to an embodiment of the present disclosure.
FIG. 6 illustrates a control block diagram of a wireless probe, according to an embodiment of the present disclosure
FIG. 7 illustrates a control block diagram of an ultrasonic imaging apparatus, according to an embodiment of the present disclosure
FIG. 8 illustrates a control block diagram of an ultrasonic imaging system including a wireless probe and an ultrasonic imaging apparatus, according to an embodiment of the present disclosure.
FIG. 9 illustrates diagrams for explaining an instance to set signal thresholds and control switching to a low-power mode based on the signal thresholds, according to an embodiment of the present disclosure
FIG. 10 illustrates a diagram for explaining an instance to derive directional information based on radiation pattern information and receive signal strength, according to an embodiment of the present disclosure.
FIG. 11 illustrates a diagram for explaining an instance to calculate position information of a wireless probe using triangulation, according to an embodiment of the present disclosure
FIG. 12 illustrates a diagram for explaining a difference between an air image and an ultrasound image including an object, according to an embodiment of the present disclosure.
FIG. 13 illustrates a flowchart for determining whether to switch into a low-power mode in various methods, according to an embodiment of the present disclosure.
FIG. 14 illustrates a flowchart illustrating operation of a wireless probe, according to an embodiment of the present disclosure.
FIG. 15 illustrates a flowchart illustrating operation of an ultrasonic imaging apparatus, according to an embodiment of the present disclosure.

### DETAILED DESCRIPTION OF EXEMPLARY EMBODIMENTS

Embodiments of the present disclosure will now be described in detail with reference to accompanying drawings.

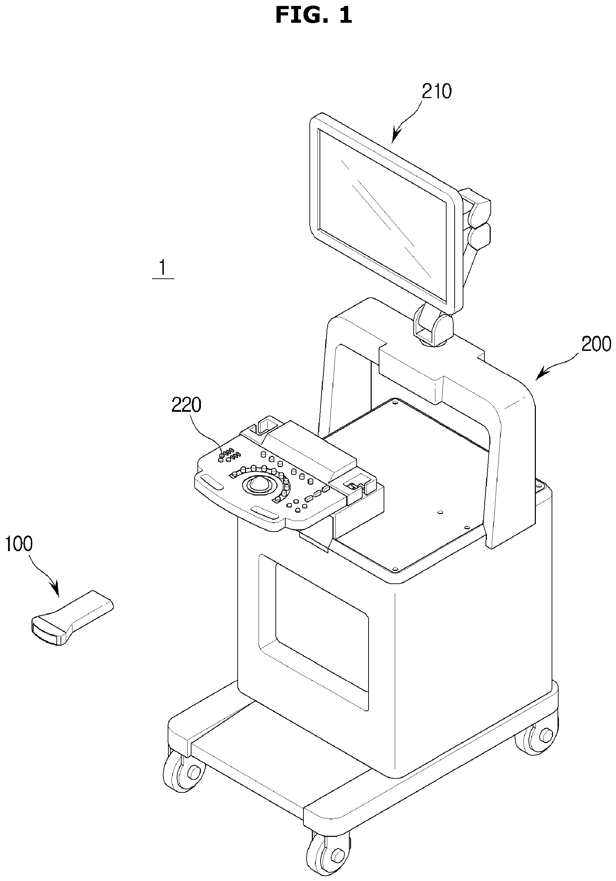

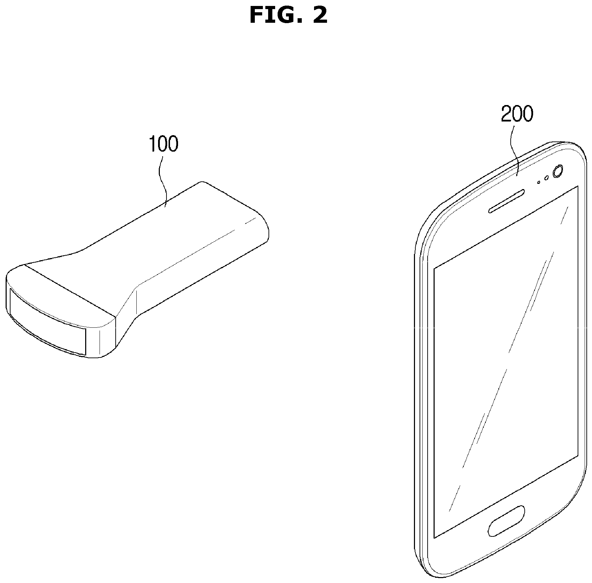

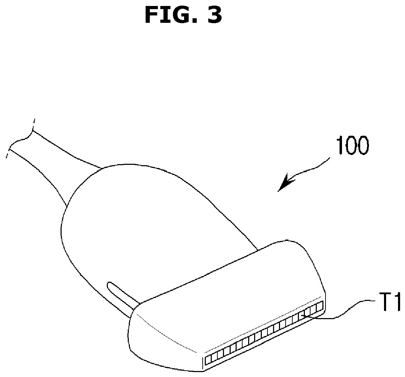

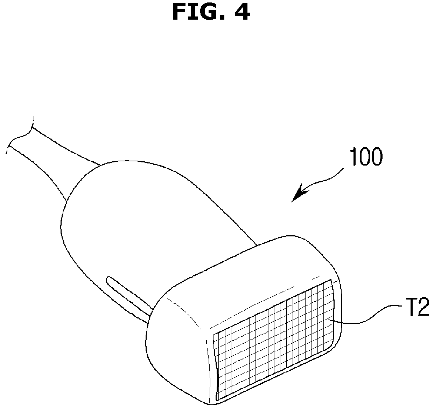

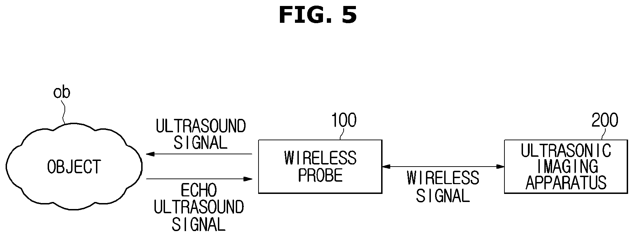

FIGS. 1 and 2 are perspective views of ultrasonic imaging systems each having a wireless probe and an ultrasonic imaging apparatus, according to different embodiments of the present disclosure. FIG. 3 illustrates an exterior view of a wireless probe including one dimensional (1D) array transducers, according to an embodiment of the present disclosure, and FIG. 4 illustrates an exterior view of a wireless probe including two dimensional (2D) array transducers, according to an embodiment of the present disclosure. FIG. 5 illustrates relations between a wireless probe and an ultrasonic imaging apparatus, according to an embodiment of the present disclosure. The following description will refer to the drawings together to prevent overlapping explanation.

Referring to FIG. 1, an ultrasonic imaging system 1 may include a wireless probe 100 for acquiring an ultrasound image by transmitting an ultrasound signal to an object, receiving an echo ultrasound signal from the object, and converting the echo ultrasound signal to an electric signal, and an ultrasonic imaging apparatus 200 connected to the wireless probe 100 via a wireless communication network for displaying the ultrasound image.

The wireless probe 100 may be connected to the ultrasonic imaging apparatus 200 over a wireless communication network for receiving various signals required to control the wireless probe 100, or for transmitting an analog or digital signal corresponding to an echo ultrasound signal received by the wireless probe 100. Furthermore, the wireless probe 100 may transmit e.g., status of operation of the wireless probe 100 to the ultrasonic imaging apparatus 200. Wireless signals as will be described below include all the aforementioned signals and all data to be transmitted/received over the wireless network.

The wireless communication network refers to a communication network that supports a wireless communication scheme for enabling wireless signal transmission/reception. For example, the wireless communication scheme may include not only a third generation (3G) or fourth generation (4G) communication scheme enabling wireless communication via a base station, but also all the communication schemes enabling wireless communication between devices located within a certain range, such as Wireless Local Area Network (WLAN), Wireless Fidelity (Wi-Fi), Bluetooth, Zigbee, Wi-Fi Direct (WFD), Ultra wideband (UWB), Infrared Data Association (IrDA), Bluetooth Low Energy (BLE), Near Field Communication (NFC), etc. The wireless communication scheme is, however, not limited thereto, and includes all the communication networks that support wireless signal communication between the wireless probe 100 and the ultrasonic imaging apparatus 200.

The ultrasonic imaging apparatus 200 may be implemented to be generally used in ultrasonic diagnosis in e.g., hospitals, as shown in FIG. 1. However, the ultrasonic imaging apparatus 200 is not limited to what is shown in FIG. 1.

For example, the ultrasonic imaging apparatus 200 may be implemented in the form of not only a laptop, desktop, tablet Personal Computer (tablet PC), but also a smart phone as shown in FIG. 2. Furthermore, the ultrasonic imaging apparatus 200 may include a mobile terminal such as a Personal Digital Assistant (PDA), and a wearable device in the form of a wearable watch or glasses.

The ultrasonic imaging apparatus 200 is not limited thereto, but may include any device implemented to have a communication unit for exchanging wireless signals with an external device over a wireless communication network and to be able to display ultrasound images on its display.

The ultrasonic imaging apparatus 200 may be configured to include an input unit 210 and a display 220, as shown in FIG. 1. The input unit 210 may receive setting information and various control commands relating to the wireless probe 100 from the user.

In an embodiment, the setting information for the wireless probe 100 may include information about gain, zoom, focus, Time Gain Compensation (TGC), depth, frequency, power, frame average, dynamic range, etc. The setting information for the wireless probe 100, however, is not limited thereto, but may include other various information that may be set to capture ultrasound images.

In another example, the input unit 210 may receive control commands for the wireless probe 100 or the ultrasonic imaging apparatus 200 from the user. The aforementioned information or commands may be delivered to the wireless probe 100 over the wired or wireless communication network, and the wireless probe 100 may then be configured based on the received information.

The input unit 210 may be implemented with a keyboard, a foot switch, or a foot pedal. For example, the keyboard may be implemented in hardware. The keyboard may include at least one of switches, keys, a joy stick, and a trackball. Alternatively, the keyboard may be implemented in software, such as a graphic user interface. In this case, the keyboard may be displayed through the main display 320. The foot switch or foot pedal may be placed in a lower part of the ultrasonic imaging apparatus 200, and the user may use the foot pedal to control operation of the ultrasonic imaging apparatus 200.

The display 220 may display an ultrasound image of a target part inside the object. The ultrasound image displayed on the display 220 may be a two dimensional (2D) or three dimensional (3D) ultrasound image, and various ultrasound images may be displayed depending on operating modes of the ultrasonic imaging apparatus 200. The display 220 may also display menus or user guides required for ultrasonic imaging, and information regarding status of operation of the wireless probe 100.

The display 220 may be implemented in various well-known display panels, such as Cathode Ray Tubes (CRTs), Liquid Crystal Displays (LCDs), Light Emitting Diodes (LEDs), Plasma Display Panels (PDPs), Organic Light Emitting Diodes (OLEDs), etc., but is not limited thereto.

In a case the display 220 is implemented in a type of touch screen, the display 220 may serve as the input unit 210 as well. In this case, the user may input various commands through the display 220 or the input unit 210.

For example, in the case of the ultrasonic imaging apparatus 200 shown in FIG. 2, e.g., virtual input buttons implemented in a graphic user interface (GUI) may be displayed on the display 220 of the ultrasonic imaging apparatus 200, and the user may input various commands with the buttons.

Features of the wireless probe 100 will be described below in more detail.

The wireless probe 100 may make contact with the surface of an object, and transmit and/or receive ultrasound signals. Specifically, under the control of the ultrasonic imaging apparatus 200, the wireless probe 100 may transmit an ultrasound signal to the inside of an object, and receive an echo ultrasound signal reflected from a particular part inside the object and forward it to the ultrasonic imaging apparatus 200. That is, the wireless probe 100 may transmit the echo ultrasound signal received from the object to the ultrasonic imaging apparatus 200 over a wireless communication network, or acquire an ultrasound image from the echo ultrasound signal and transmit the ultrasound image, but is not limited thereto.

The wireless probe 100 may include a transducer array for performing conversion between electric and ultrasound signals to transmit ultrasounds into the object or receive an echo ultrasound signal from the object. The transducer array may include a plurality of transducer elements.

A transducer module as will be described below may include a transducer array that has a plurality of transducer elements. The transducer array may be 1D array or 2D array. In an embodiment, the transducer module may include 1D array transducers T1 as shown in FIG. 3. In another embodiment, the transducer module may include 2D array transducers T2 as shown in FIG. 4.

For example, the 1D array transducers include transducer elements, each of which may convert between ultrasound signals and electric signals. For this, the transducer elements may be implemented with magnetostrictive ultrasonic transducers that use magnetostrictive effects of a magnetic substance, piezoelectric ultrasonic transducers that use piezoelectric effects of a piezoelectric material, piezoelectric micromachined ultrasonic transducers (pMUTs), or the like, or may also be implemented with capacitive micromachined ultrasonic transducers (cMUTs) that transmit/receive ultrasounds using vibration of hundreds or thousands of micromachined thin films.

The transducers may also be arrayed linearly, or convexly. The basic operating principle of the wireless probe 100 may be equally applied to both cases, but in the case of the wireless probe 100 having transducers convexly arrayed, ultrasounds irradiated from the transducers are fan-shaped, thus forming a fan-shaped ultrasound image.

Referring to FIG. 4, the transducer module may include 2D array transducers T2 as described above. With the 2D array transducers T2, the internal part of the object may be imaged in 3D.

Each of the transducer elements included in the 2D transducer array is the same as that of the 1D transducer array, so the description will be omitted herein. Relations between the wireless probe 100 and the ultrasonic imaging apparatus 200 will now be described.

FIG. 5 illustrates relations between a wireless probe and an ultrasonic imaging apparatus, according to an embodiment of the present disclosure.

An ultrasonic system may include the wireless probe 100 and the ultrasonic imaging apparatus 200. Referring to FIG. 5, the ultrasonic system uses the wireless probe 100 to irradiate an ultrasound signal from the surface of an object (ob) to a target part inside the object, and uses the reflected ultrasound signal, i.e., echo ultrasound signal to obtain and provide an image of a cross-section of soft tissue or blood flow for the user in a non-invasive method.

In the ultrasonic imaging apparatus 200, there may be an image processor for converting the received echo ultrasound signal to an ultrasound image. The image processor may be implemented in hardware, such as a microprocessor, or in software that may be carried out in the hardware.

Alternatively, an image processor may be built into the wireless probe 100 to convert the echo ultrasound signal to the ultrasound image. The wireless probe 100 may then send the ultrasound image to the ultrasonic imaging apparatus 200 over a wireless communication network, but is not limited thereto. The image processor may obtain image information from the ultrasound image. This will be described in more detail later.

In the meantime, the ultrasound image created by the image processor may be stored in a memory unit in the ultrasonic imaging apparatus 200. Alternatively, the ultrasound image may be stored in a Web storage or cloud server having a storage function on the Web. Features of the wireless probe and ultrasonic imaging apparatus will now be described in more detail.

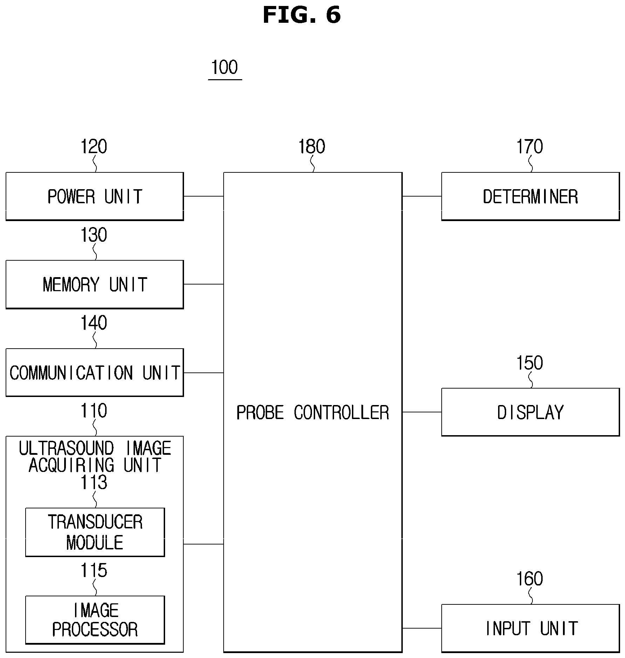

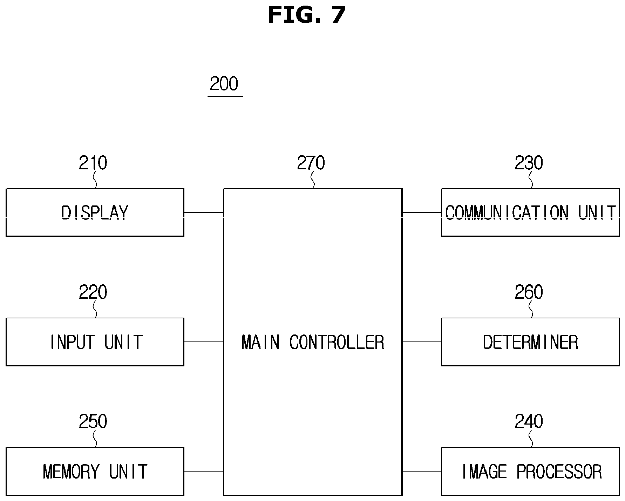

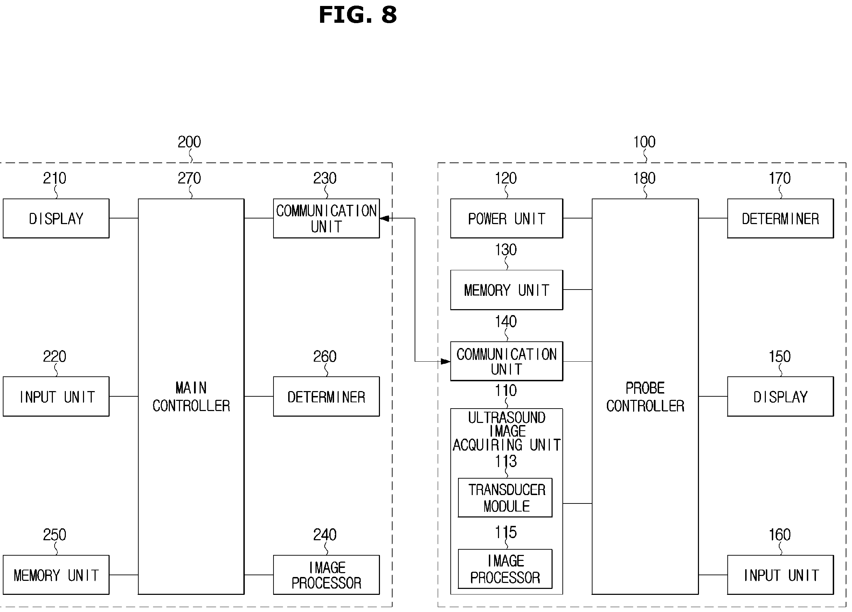

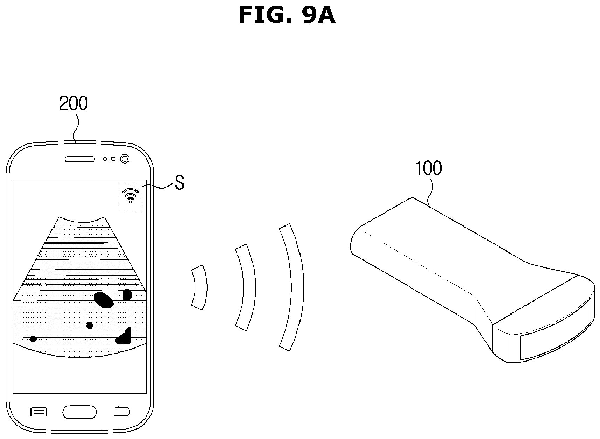

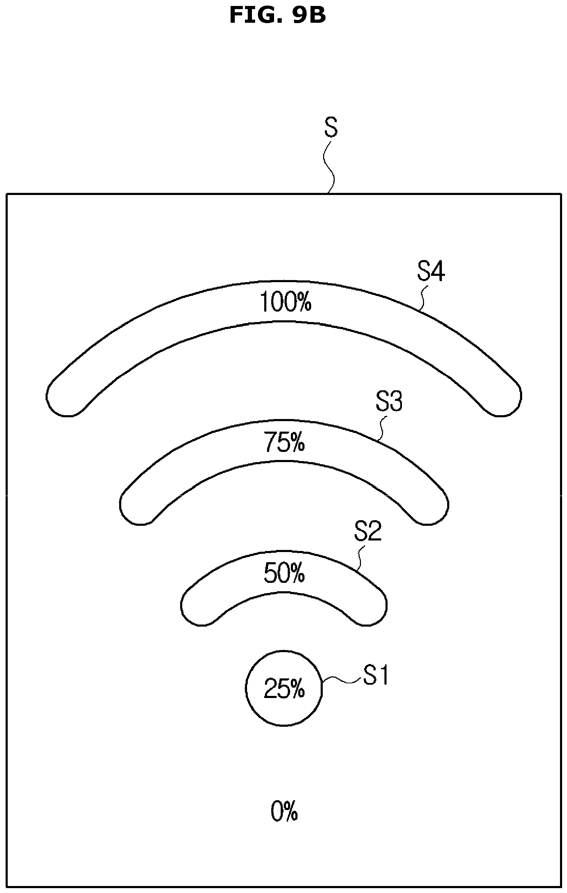

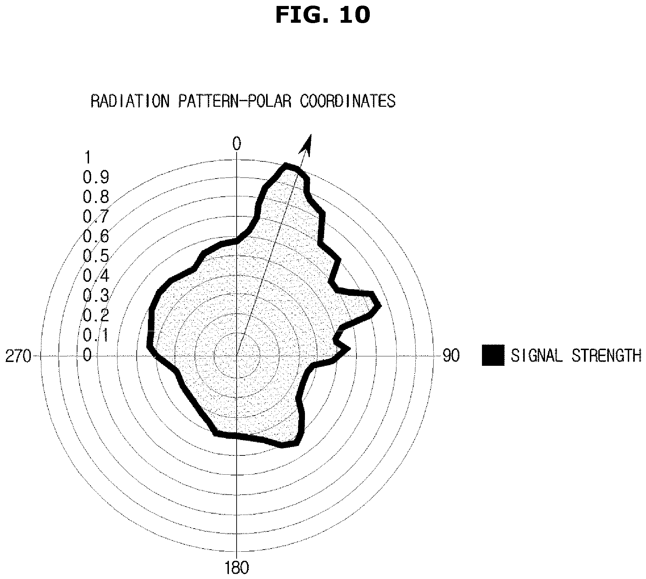

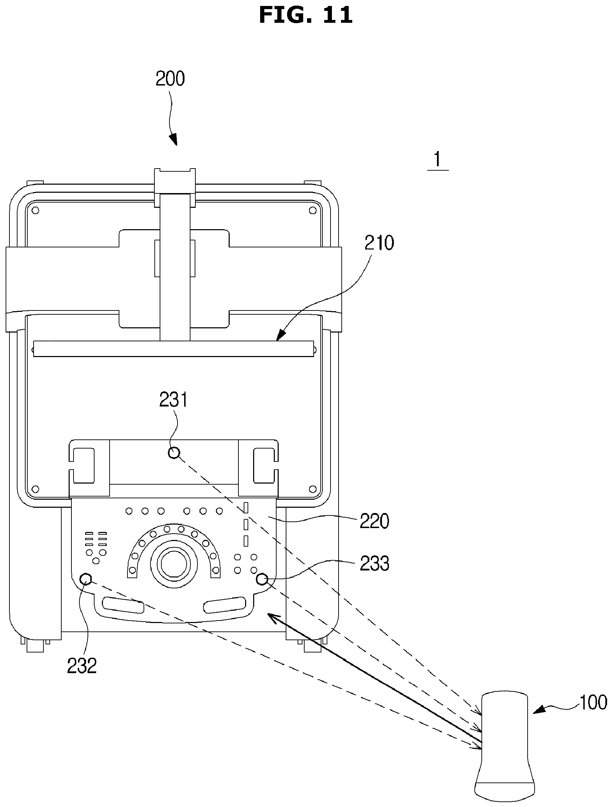

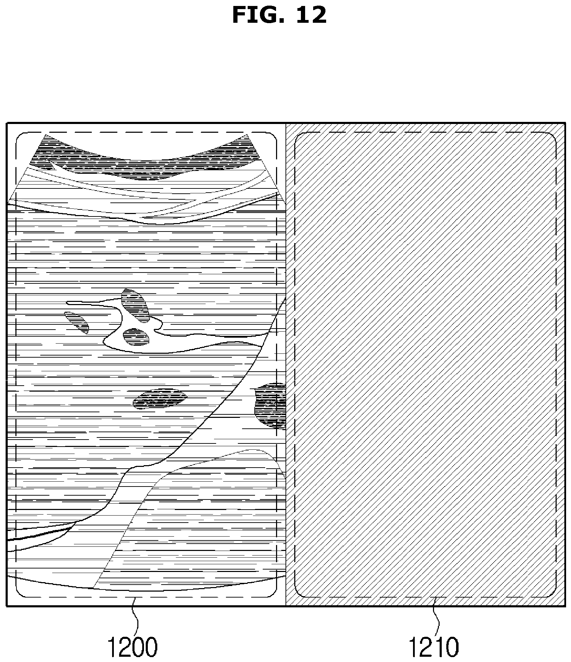

FIG. 6 is a control block diagram of a wireless probe, according to an embodiment of the present disclosure, FIG. 7 is a control block diagram of an ultrasonic imaging apparatus, according to an embodiment of the present disclosure, and FIG. 8 is a control block diagram of an ultrasonic imaging system including a wireless probe and an ultrasonic imaging apparatus, according to an embodiment of the present disclosure. FIG. 9 illustrates diagrams for explaining an instance to set signal thresholds and control switching to a low-power mode based on the signal thresholds, according to an embodiment of the present disclosure, and FIG. 10 illustrates a diagram for explaining an instance to derive directional information based on radiation pattern information and receive signal strength, according to an embodiment of the present disclosure. FIG. 11 illustrates a diagram for explaining an instance to calculate position information of a wireless probe using triangulation, according to an embodiment of the present disclosure, and FIG. 12 illustrates a diagram for explaining a difference between an air image and an ultrasound image including an object, according to an embodiment of the present disclosure. The following description will refer to the drawings together to prevent overlapping explanation.

FIG. 6 is a block diagram of a wireless probe and an ultrasonic imaging apparatus, according to an embodiment of the present disclosure.

Referring to FIG. 6, the wireless probe 100 may include an ultrasound image acquiring unit 110, a power unit 120, a memory unit 130, a communication unit 140, a display 150, an input unit 160, a determiner 170, and a probe controller 180. The ultrasonic image acquiring unit 110 may include a transducer module 113 and an image processor 115.

At least one of the image processor 115, the memory unit 130, the communication unit 140, the determiner 170, and the probe controller 180 may be integrated in a system on chip (SoC) built into the wireless probe 100. It is not limited as being integrated in one SoC because the wireless probe 100 may have more than one SoCs.

The ultrasound image acquiring unit 110 may transmit ultrasound signals, and acquire ultrasound images. Referring to FIG. 6, the ultrasonic image acquiring unit 110 may include the transducer module 113 and the image processor 115.

The transducer module 113 may transmit an ultrasound signal to an object, and receive an echo ultrasound signal reflected from the object. The transducer module 113 is the same as what is described above, so the description will be omitted herein.

The image processor 115 may acquire an ultrasound image from the echo ultrasound signal. For example, the image processor 115 may acquire ultrasound images in many different modes, such as amplitude mode (A mode), brightness mode (B mode), motion mode (M mode), Doppler mode, etc.

In the case the ultrasonic probe 100 includes the 2D array transducers or 1D array transducers that may be driven in the elevational direction, the image processor 115 may even create a 3D ultrasound image with a plurality of 2D cross-sectional images.

The image processor 115 may obtain image information from the ultrasound image through image processing as will be described below. The image information may be information about pictures contained in the respective pixels in the ultrasound image, including luminosity, brightness, color, illuminance, chroma, etc. Accordingly, the determiner 170 may determine whether to switch into the low-power mode by comparing the image information obtained from the ultrasound image with an air image in a mode corresponding to the ultrasound image. This will be described in more detail later.

The power unit 120 may supply power to the wireless probe 100. Specifically, the power unit 120 may convert electric energy to chemical energy, accumulate the chemical energy, and convert the accumulated chemical energy to electric energy to supply power. In an embodiment, the power unit 120 may be implemented by a lithium-ion battery, nickel hydrate battery, polymer battery, etc. The power unit 120, however, is not limited thereto, but may be implemented in various types of batteries built into the wireless probe 100 for supplying power.

The power unit 120 may be charged wirelessly, or charged by directly contacting a charging device. That is, the power unit 120 may be charged in any method known to the public without limitations.

The memory unit 130 may store various data. For example, the memory unit 130 may store data about ultrasound images acquired by the image processor 115, image information obtained from the ultrasound image, etc.

Furthermore, the memory unit 130 may store data about air images in respective modes. The air image refers to an ultrasound image acquired when an ultrasound signal is reflected from air.

For example, to acquire an ultrasound image of an object through the wireless probe 100, a water-soluble gel is applied onto the transducer array. For example, impedance of air is about 0.0004Ω, and impedance of water is about 1.48Ω. Furthermore, reflectivity of air is about 99.9%, and reflectivity of water is about 0.2326%.

Specifically, the transducer module 113 may include a piezoelectric layer that converts between electric and acoustic signals through vibration of a piezoelectric material, an acoustic matching layer that reduces a difference in acoustic impedance between the piezoelectric layer and an object such that an ultrasound produced in the piezoelectric layer may be delivered to the object as much as possible, a lens layer that focuses the ultrasound propagating forward of the piezoelectric layer onto a particular point, and a sound-absorption layer that cuts off the ultrasound from propagating backward of the piezoelectric layer to prevent image distortion.

Accordingly, there is a significant difference between the impedance inside the transducer module 113 and the impedance of air, and thus, the transducer module 113 may not be able to receive echo ultrasound signals due to the significant difference in impedance between the air and the inside of the transducer module 113.

For example, referring to FIG. 12, an ultrasound image 1200 including an object may differ from an air image 1210. Accordingly, the determiner 170 may determine whether to switch into the low-power mode by comparing the air image data and the acquired ultrasound image.

The memory unit 130 may be implemented with at least one type of storage media, such as flash memories, hard disks, multimedia card micro type memories, card type memories (e.g., SD or XD memories), Random Access Memories (RAMs), Static Random Access Memories (SRAMs), Read-Only Memories (ROMs), Electrically Erasable Programmable Read-Only Memories (EEPROMs), Programmable Read-Only Memories (PROMs), magnetic memories, magnetic disks, and optical disks. However, the memory unit 130 is not limited thereto, but may be implemented in any other form known in the art.

The communication unit 140 is configured to exchange various data with the ultrasonic imaging apparatus 200 over a wireless communication network. The communication unit 140 may transmit and/or receive an ultrasound image of an object, data relating to diagnosis of the object, such as echo ultrasounds, Doppler data, etc., over the wireless communication network. Furthermore, the communication unit 140 may receive various control command signals from the ultrasonic imaging apparatus 200. There are not limitations on types of data or commands that the communication unit 140 may exchange in radio signals.

The communication unit 140 may include one or more components enabling wireless communication with other external electronic devices. For example, the communication unit 140 may perform wireless communication with the ultrasonic imaging apparatus 200 through at least one of a short-range communication module and a mobile communication module.

The short-range communication module refers to a module for near distance communication within a predetermined range. For example, the short-range communication may include Wireless Local Area Network (WLAN), Wi-Fi, Bluetooth, Zigbee, Wi-Fi Direct (WFD), Ultra Wideband (UWB), Infrared Data Association (IrDA), Bluetooth Low Energy (BLE), Near Field Communication (NFC), etc., but is not limited thereto.

The mobile communication module may transmit and receive Radio Frequency (RF) signals to and from one of base stations, external terminals, and servers in the mobile communication network. For example, the mobile communication module may exchange various types of data with the ultrasonic imaging apparatus 200 via a base station over 3G or 4G communication networks. Hereinafter, the short range communication module and the mobile communication module are collectively called a communication module.

For example, the communication unit 140 may include a plurality of communication modules. The plurality of communication modules may be located at different positions. Accordingly, the determiner 170 may determine whether to switch into the low-power mode by obtaining position information between the wireless probe 100 and the ultrasonic imaging apparatus 200 based on signals received through the communication modules located at different positions. This will be described in more detail later.

The wireless probe 100 may include the display 150. The display 150 may be implemented by various display panels known to the public, such as a CRT, an LCD, LEDs, a PDP, OLEDs, etc., as described above, but is not limited thereto. the display 150 may display information relating to status of operation of the wireless probe 100, such as power status of the wireless probe 100.

Furthermore, the wireless probe 100 may include the input unit 160. The input unit 160 may be implemented in the form of switch(es), key(s), etc., as described above, but is not limited thereto. The input unit 160 may receive power on/off commands, control commands for operation mode changes, etc., for the wireless probe 100 from the user without limitations.

In the meantime, the wireless probe 100 may include the determiner 170 as shown in FIG. 6.

The determiner 170 may determine whether to switch into the low-power mode based on at least one of a condition of a wireless communication network, whether an ultrasound image contains an object, position information between the ultrasonic imaging apparatus 200 and the wireless probe 100, and whether waiting time has passed.

The condition of a wireless communication network refers to a condition of connection between the wireless prove 100 and the ultrasonic imaging apparatus 200 over the wireless communication network. The condition of the wireless communication network may be determined based on various parameters.

For example, the condition of the wireless communication network may be determined based on various parameters used as references for connection condition, such as transfer rate, signal quality, signal to noise ratio (SNR), and receive signal strength indication (RSSI), etc., of wireless signals over the wireless communication network, and based on various other parameters known to the public.

The signal quality may be determined based on whether waveform, amplitude, distortion, etc., of the received signal is within acceptable limits of fluctuation, or based on an amount of fluctuation of the received signal. The SNR may be determined based on the magnitude of the signal to noise ratio.

The determiner 170 may determine whether the wireless communication network is in an appropriate condition for acquiring and providing an ultrasound image for the user, based on the condition of the wireless communication network.

The determiner 170 may determine whether to switch into the low-power mode by comparing the condition of the wireless communication network with a predetermined condition. For example, the determiner 170 may compare the condition of the wireless communication network with a predetermined signal threshold, and determine to switch into the low-power mode if receive sensitivity over the wireless communication network is lower than the predetermined signal threshold.

The signal threshold refers to a threshold of the condition of the wireless communication network. The signal threshold may include a threshold of receive sensitivity, a threshold of receive signal strength, a threshold of transfer rate, and any other thresholds set for various parameters. Accordingly, whether to switch into the low-power mode may be determined based on whether the condition of the wireless communication network is above the signal threshold.

The information about the signal threshold may be set and stored in advance. In an embodiment, information about the signal threshold may be set in advance in a design stage and stored in the memory 130. Alternatively, the information about the signal threshold may be received from the user and stored in the memory 130.

The user may input information about the signal threshold through the input unit 220 of the ultrasonic imaging apparatus 200 or the input unit 160 of the wireless probe 100. For example, if the wireless communication corresponds to Wi-Fi communication, a Wi-Fi status bar S may be displayed on the display 210 of the ultrasonic imaging apparatus 200, as shown in FIG. 9A.

It may be interpreted that the more the status bar is filled, the higher the receive sensitivity is. For example, referring to FIG. 9B, if a first status bar S1 is lighted, it means that the receive sensitivity is about 25%. Further, if the first and second status bars S1 and S2 are lighted, it means that the receive sensitivity is about 50%. Moreover, if the first to third status bars S1, S2, and S3 are lighted, it means that the receive sensitivity is about 75%. Furthermore, if the first to fourth status bars S1, S2, S3, and S4 are lighted, it means that the receive sensitivity is about 100%.

For example, if the transfer rate of the wireless signal is lower than a predetermined transfer rate, the determiner 170 may determine that it is difficult to transmit an ultrasound image or echo ultrasound signal to the ultrasonic imaging apparatus 200 over the wireless communication network to provide it to the user in real time, and determine to switch to the low-power mode from a scanning mode.

In another example, if the signal quality of the wireless signal is lower than a predetermined signal quality, the determiner 170 may determine that it is difficult to transmit an ultrasound image or echo ultrasound signal to the ultrasonic imaging apparatus 200 over the wireless communication network to provide it to the user in real time, and determine to switch to the low-power mode from a scanning mode.

Furthermore, the determiner 170 may determine whether to switch into the low-power mode by determining whether the ultrasound image includes the object using image information obtained through image processing on the ultrasound image.

As described above, the memory unit 130 may store information about air images in respective modes. Accordingly, the determiner 170 may determine whether the ultrasound image includes the object by comparing the ultrasound image and an air image in a mode corresponding to the ultrasound image.

For example, the determiner 170 may determine similarity between the ultrasound image and an air image in the same mode as for the ultrasound image using image information, and determine to switch into the low-power mode if the images are similar to each other more than a predetermined range, which means that the object is not included in the ultrasound image.

Furthermore, the determiner 170 may determine whether to switch into the low-power mode according to whether air images are acquired continuously for a predetermined waiting time. For example, the determiner 170 may determine whether the user has an intent to use the wireless probe 100 according to whether the air image has been created for a certain period of time, thereby preventing the wireless probe 100 from being switched into the low-power mode even if the user wants to scan the object to acquire its ultrasound image. The information about the waiting time may be set by the user or set in a design stage, and stored in the memory 130.

In addition, the determiner 170 is configured to determine whether to switch into the low-power mode using position information between the wireless probe 100 and the ultrasonic imaging apparatus 200. The position information includes directional information and distance information between the wireless probe 100 and the ultrasonic imaging apparatus 200.

The distance information between the wireless probe 100 and the ultrasonic imaging apparatus 200 may be determined based on receive signal strength. For example, the strength of a wireless signal received from the ultrasonic imaging apparatus 200 is inversely proportional to the distance between the wireless probe 100 and the ultrasonic imaging apparatus 200. Specifically, the longer the distance between the wireless probe 100 and the ultrasonic imaging apparatus 200, the weaker the strength of the received wireless signal. On the other hand, the shorter the distance between the wireless probe 100 and the ultrasonic imaging apparatus 200, the stronger the strength of the received wireless signal. There are no limitations on the signal used in calculating the receive signal strength.

In addition, the determiner 170 may calculate the directional information between the wireless probe 100 and the ultrasonic imaging apparatus 200 using radiation pattern information. Referring to FIG. 10, shown is a radiation pattern of radiation power being radiated from an antenna of the ultrasonic imaging apparatus 200 as signals are transmitted/received over a wireless communication network. Specifically, the radiation power refers to power radiated as the antenna radiates the signals, and the radiation pattern is represented as a directional function of the radiation power from the antenna of the ultrasonic imaging apparatus 200, which is produced as the wireless signals are exchanged between the wireless probe 100 and the ultrasonic imaging apparatus 200.

The determiner 170 may determine a direction in which the ultrasonic imaging apparatus 200 is located, using the radiation pattern information. As shown in FIG. 10, signal strength may be strong in the direction in which the wireless probe 100 and the ultrasonic imaging apparatus 200 exchange signals. For example, the ultrasonic imaging apparatus 200 may be situated at the center of the radiation pattern while the wireless probe 100 is located in the direction having the highest signal strength in the radiation pattern.

In another example, the determiner 170 may derive the directional information between the wireless probe 100 and the ultrasonic imaging apparatus 200 using triangulation. The triangulation is a method for estimating position information between devices on a 2D plane, and requires at least three or more reference points. For this, the wireless probe 100 may have three communication modules located at different positions.

Accordingly, the determiner 170 may figure out information about distances between the respective communication modules and the ultrasonic imaging apparatus 200 based on receive signal strengths with respect to the respective communication modules, and determine that the ultrasonic imaging apparatus 200 is located at a point of contact on the coordinate system based on the distance information, thereby deriving directional information of the ultrasonic imaging apparatus 200 as well.

In another example, the wireless probe 100 may include at least one position measurement sensor. The position measurement sensor refers to a sensor for measuring a position of the wireless probe 100.

For example, the position measurement sensor may include, but not exclusively, a Global Positioning System (GPS) for measuring the position with satellites, and a Differential Global Positioning System (DGPS) that is capable of measuring a position with high precision by supplementing the GPS.

Position information (or location information) transmitted to a terrestrial GPS from a satellite often has an error. For example, if there are N GPSs, where NS2, located close to one another, the N GPSs may have similar errors. In this regard, DGPS may obtain more accurate data by offsetting common errors that the N GPSs have to each other.

The position measurement sensor may figure out a position of the wireless probe 100. Furthermore, as will be described later, the ultrasonic imaging device 200 may also include a position measurement sensor to measure a position of the ultrasonic imaging apparatus 200. In this case, the determiner 170 may determine whether to switch into the low-power mode by comparing the position information of the ultrasonic imaging apparatus 200 received from the ultrasonic imaging apparatus 200 with the position information of the wireless probe 100.

For example, if a distance between the wireless probe 100 and the ultrasonic imaging apparatus 200 is not greater than a predetermined distance, the determiner 170 may determine that ultrasound scanning is underway, thus determining not to switch into the low-power mode. In other words, if it is determined that the user is currently performing ultrasound scanning on an object because the distance between the ultrasonic imaging apparatus 200 and the wireless probe 100 is close, the determiner 170 may determine to remain in the current scanning mode. The predetermined distance may be set in advance in a design stage, or set by the user.

On the other hand, if the distance between the wireless probe 100 and the ultrasonic imaging apparatus 200 is greater than the predetermined distance and a direction between the wireless probe 100 and the ultrasonic imaging apparatus 200 is typically determined to hardly appear in ultrasound image scanning, the determiner 170 may determine that ultrasound scanning is not underway, thus determining to switch into the low-power mode.

For example, in ultrasound image scanning on an object, the user performs the scanning while checking the ultrasound image in real time. Accordingly, if the wireless probe 100 is located opposite to the frontal direction of the ultrasonic imaging apparatus 200, it is determined that the ultrasound image scanning is not underway. The determiner 170 is configured to determine that the ultrasound image scanning is not underway, thus determining to switch into the low-power mode.

Switching into the low-power mode may be determined not only when the wireless probe 100 is located opposite to the frontal direction of the display 220 of the ultrasonic imaging apparatus 200.

For example, even if the wireless probe 100 is located in front of the display 220 of the ultrasonic imaging apparatus 200, if an angle between the wireless probe 100 and the display 220 of the ultrasonic imaging apparatus 200 is out of a predetermined range, it is difficult for the user to perform ultrasound scanning while watching the display 220. Accordingly, the determiner 170 may determine whether to switch into the low-power mode according to whether the angle between the wireless probe 100 and the display 220 of the ultrasonic imaging apparatus 200 is out of the predetermined range.

The probe controller 180 may control general operation of the wireless probe 100. The probe controller 180 may be implemented by a processor built into the ultrasonic probe 100, and generate control signals to control respective operation of the aforementioned components.

For example, the probe controller 180 may control the ultrasound image acquiring unit 110 with a control signal to control transmission of an ultrasound signal and reception of an echo ultrasound signal. In another example, the probe controller 180 may control the communication unit 140 with a control signal to perform communication with an external device.

In yet another example, once it is determined to switch into the low-power mode, the probe controller 180 may control the power unit 120 with a control signal to control supply of power. Specifically, if it is determined that ultrasound image scanning is not under way or that it is not in a normal condition to provide the ultrasound image to the user, the probe controller 180 may control the power unit 120 to prevent unnecessary power consumption.

In another embodiment, the probe controller 180 may control switching into the low-power mode according to a determination result based on at least one of a condition of a wireless communication network, whether an ultrasound image includes an object, position information between the ultrasonic imaging apparatus 200 and the wireless probe 100, and whether the waiting time has passed. Even while an ultrasound scanning mode remains, if the user has no intent to perform the ultrasound image scanning, i.e., if ultrasound scanning is not currently underway, unnecessary power is consumed to maintain the scanning mode.

In an embodiment, if it is determined that the object is not contained in the ultrasound image that has been obtained until the waiting time has passed even in the scanning mode of the wireless probe 100, the probe controller 180 may control the wireless probe 100 to be switched into the low-power mode.

Furthermore, if the user may not normally check the ultrasound image through the ultrasonic imaging apparatus 200 due to poor conditions of the wireless communication network, even if scanning is being actually performed, it has no meaning.

Accordingly, in an embodiment, the wireless probe 100 may collect various information to determine whether the user actually has an intent to perform ultrasound image scanning, and combine them to determine whether to switch into the low-power mode. In other words, the wireless probe 100 may provide both user convenience and power management efficiency by more precisely figuring out a need to switch into the low-power mode.

The ultrasonic imaging apparatus 200 will now be described in more detail.

Referring to FIG. 7, the ultrasonic imaging apparatus 200 may include a display 210, an input unit 220, a communication unit 230, an image processor 240, a memory unit 250, a determiner 260, and a main controller 270. At least one of the communication unit 230, the image processor 240, the memory unit 250, the determiner 260, and the main controller 270 may be integrated in a SoC built into the ultrasonic imaging apparatus 200. It is not limited as being integrated in one SoC because the ultrasonic imaging apparatus 200 may have more than one SoCs.

The display 210 may display various information. The display is configured to display an ultrasound image acquired through image processing on an echo ultrasound signal. If the display 210 is implemented in a type of touch screen, the display 210 may display a GUI for the user to input various control commands for the ultrasonic imaging apparatus 200 and the wireless probe 100. The display 210 implemented in the type of touch screen may also serve as the input unit 220.

The communication unit 230 may exchange data with an external device over a communication network. The communication network includes a wired communication network in addition to the aforementioned wireless communication network. The wireless communication network will not be described because it was already described above.

The wired communication network refers to a communication network that enables wired communication of signals including data. For example, the wired network may include Peripheral Component Interconnect (PCI), PCI-express, Universe Serial Bus (USB), etc., but is not limited thereto. That is, the communication unit 230 may exchange wireless signals including various data with the communication unit 140 of the wireless probe 100 over a wireless communication network, and also exchange signals including various data with an external device via a wired communication network.

The image processor 240 may acquire an ultrasound image from an echo ultrasound signal, or obtain image information by performing image processing on the ultrasound image. For example, the image processor 240 may acquire an ultrasound image from an echo ultrasound signal received from the wireless probe 100 through the communication unit 230. The image information is the same as what is described above, so the description will be omitted.

The ultrasonic imaging apparatus 200 may include a memory unit 250.

The memory unit 250 may store various data. For example, the memory unit 250 may store data about ultrasound images, image information obtained from the ultrasound image, etc. Furthermore, the memory unit 250 may store data about air images in respective modes.

The memory unit 250 may be implemented in the type of flash memory, hard disk, multimedia card, micro memory, card memory (e.g., SD or XD memory), etc., but is not limited thereto.

The ultrasonic imaging apparatus 200 may further include a determiner 260.

The determiner 260 may determine whether to switch the wireless probe 100 into the low-power mode based on at least one of a condition of a wireless communication network supporting wireless communication with the wireless probe 100, whether the ultrasound image includes an object, position information between the ultrasonic imaging apparatus 200 and the wireless probe 100, and whether the waiting time of the wireless probe 100 has passed.

As described above, the communication unit 230 may exchange wireless signals including various data with the communication unit 140 of the wireless probe 100 over a wireless communication network. Accordingly, the communication unit 230 may deliver a control command for the wireless probe 100, and in response, the communication unit 140 of the wireless probe 100 may deliver various data, such as data about processing results in response to the control command, data about status of operation of the wireless probe 100, etc.

For example, the determiner 260 may check status of connectivity of the wireless communication network that supports connection with the wireless probe 100, and determine whether to switch into the low-power mode based on the checking result. The determiner 260 may determine whether to switch into the low-power mode by checking the condition in which wireless signals are exchanged with the wireless probe 100 through the communication network 230.

In this regard, the determiner may determine the condition of the network communication network based on transfer rate, signal quality, SNR, receive signal strength, etc., of the wireless signals over the wireless communication network, and in addition, using various parameters known to the public.

The determiner 250 may determine whether it is appropriate to provide the ultrasound image to the user based on the condition of the wireless communication network supporting wireless communication with the wireless probe 100. How to determine whether to switch into the low-power mode based on the condition of the wireless communication network is the same as what is described in connection with the wireless probe 100, so the detailed description will be omitted herein.

Furthermore, the determiner 260 may determine whether to switch into the low-power mode by determining whether the ultrasound image includes an object using image information obtained through image processing on the echo ultrasound image.

The ultrasound image may be acquired by the image processor 240 of the ultrasonic imaging apparatus 200. Alternatively, the ultrasound image may be acquired by the image processor 115 of the wireless probe 100 and transmitted over the wireless communication network. The image information may be obtained by the image processor 240 of the ultrasonic imaging apparatus 200 or obtained and transmitted by the image processor 115 of the wireless probe 100.

For example, the determiner 260 may determine similarity between the ultrasound image and an air image in the same mode as for the ultrasound image by pixel-by-pixel comparison of image information, and determine whether the ultrasound image includes an object based on the determination of similarity. In an embodiment, if the similarity determined by pixel-by-pixel comparison of image information between the ultrasound image and the air mode in the same mode as for the ultrasound image is higher than a predetermined level, the determiner 260 may determine to switch the wireless probe 100 into the low-power mode.

Furthermore, the determiner 250 may determine whether to switch into the low-power mode according to whether air images are acquired continuously for a predetermined waiting time. For example, the determiner 250 may determine whether the user has an intent to use the wireless probe 100 according to whether air images have been created for a certain period of time, thereby preventing the wireless probe 100 from being switched into the low-power mode even if the user wants to scan the object to acquire its ultrasound images.

In addition, the determiner 250 may determine whether to switch into the low-power mode using position information between the wireless probe 100 and the ultrasonic imaging apparatus 200. In this case, the determiner 250 may determine whether to switch into the low-power mode using position information of the ultrasonic imaging apparatus 200 measured through the position measurement sensor and position information of the wireless probe 100 received from the wireless probe 100.

In addition, the determiner 250 may determine whether to switch into the low-power mode by measuring position information between the wireless probe 100 and the ultrasonic imaging apparatus 200 using receive signal strength and radiation pattern information. The receive signal strength and radiation pattern information are the same as what are described above in connection with the wireless probe 100, so the description will be omitted. That is, details of the receive signal strength and radiation pattern information are the same as described above except that an entity to calculate the receive signal strength and radiation pattern information is changed to the ultrasonic imaging apparatus 200 from the wireless probe 100. In this case, the wireless probe 100 may be situated at the center of the radiation pattern while the ultrasonic imaging apparatus 200 is located in the direction having the highest signal strength in the radiation pattern.

Furthermore, the ultrasonic imaging apparatus 100 may have three communication modules located at different positions. Accordingly, the determiner 260 may figure out information about distances between the respective communication modules and the wireless probe 100 based on receive signal strengths with respect to the respective communication modules, and determine that the wireless probe 100 is located at a point of contact on the coordinate system based on the distance information.

For example, referring to FIG. 11, the communication unit 230 of the ultrasonic imaging apparatus 100 may include three communication modules 231, 232, and 233. The three communication modules 231, 232, and 233 may be located distant to one another, as shown in FIG. 11.

The determiner 260 may figure out information about distances between the respective communication modules 231, 232, and 233 and the wireless probe 100 based on receive signal strengths calculated by the respective communication modules 231, 232, and 233, and determine that the wireless probe 100 is located at a point of contact on the coordinate system based on the distance information, thereby deriving directional information between the wireless probe 100 and the ultrasonic imaging apparatus 200 as well.

For example, if a distance between the wireless probe 100 and the ultrasonic imaging apparatus 200 is not greater than a predetermined distance, the determiner 260 may determine that ultrasound scanning is underway, thus determining not to switch into the low-power mode. In other words, if it is determined that the user is currently performing ultrasound scanning on an object because the distance between the ultrasonic imaging apparatus 200 and the wireless probe 100 is close, the determiner 260 may determine to remain in the current scanning mode. A distance used as a threshold to switch into the low-power mode may be set in advance in a design stage or set by the user, and stored in the memory unit 250.

On the other hand, if the distance between the wireless probe 100 and the ultrasonic imaging apparatus 200 is greater than the predetermined distance and a direction between the wireless probe 100 and the ultrasonic imaging apparatus 200 is typically determined to hardly appear in ultrasound image scanning, the determiner 260 may determine that ultrasound scanning is not underway or that the user has no intent to use the wireless probe 100, thereby determining to switch the wireless probe 100 into the low-power mode.

The ultrasonic imaging apparatus 200 may include a main controller 260. The main controller 260 may be implemented by a device capable of doing various operations, such as a processor.

The main controller 260 may generate control signals to control the respective components of the ultrasonic imaging apparatus 200. For example, upon reception of a control command from the user through the input unit 220, the main controller 260 may generate a control signal corresponding to the control command to control at least one of the components of the ultrasonic imaging apparatus 200.

In an embodiment, the main controller 260 may control the communication unit 230 with a control signal to transmit a switching command signal to the wireless probe 100. The switching command signal refers to a signal including a command to switch the wireless probe 100 into the low-power mode.

Specifically, if the determiner 250 determines that switching into the low-power mode is required, based on the status of the wireless probe 100, the main controller 260 is configured to control the communication unit 230 to send the switching command signal to control operation of the wireless probe 100. Accordingly, the ultrasonic imaging apparatus 200 may prevent unnecessary power consumption of the wireless probe 100.

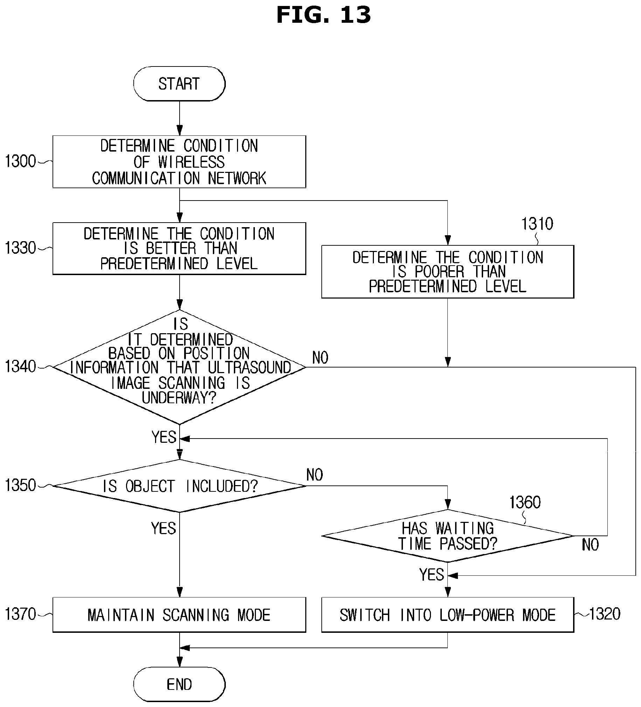

FIG. 13 is a flowchart for determining whether to switch into a low-power mode in various methods, according to an embodiment of the present disclosure.

An ultrasonic imaging system may capture an ultrasound image through the wireless probe, and provide the ultrasound image to the user through the ultrasonic imaging apparatus. In this case, it is important to manage power consumption of the wireless probe due to miniaturization of the wireless probe. Accordingly, even when the user has turned on the wireless probe, if scanning is not underway, i.e., if it is determined that the user is not currently using the wireless probe, it is important to reduce power consumption of the wireless probe by switching the wireless probe into the low-power mode. A method for determining whether to switch to the low-power mode from the scanning mode will now be described.

An entity to determine whether to switch into the low-power mode may be one of the ultrasonic imaging apparatus and the wireless probe, which constitute the ultrasonic imaging system.

The ultrasonic imaging system may determine a condition of a wireless communication network between the ultrasonic imaging apparatus and the wireless probe, in operation 1300. The condition of the wireless communication network refers to connection status of the wireless communication network, which may be determined based on transfer rate, signal quality, SNR, and receive signal strength of the wireless signal.

If the condition of the wireless communication network is determined to be appropriate for the wireless probe to transmit ultrasound images or echo ultrasound signals for the ultrasonic imaging apparatus to provide them to the user in real time, the ultrasonic imaging system may determine that the condition of the wireless communication network is appropriate for use, thereby remaining in the scanning mode.

If the condition is poorer than a predetermined condition in operation 1310, the ultrasonic imaging system may determine that the wireless probe is not able to provide the scanned ultrasound image to the user in real time, thereby leading the wireless probe to be switched into the low-power mode.

For example, if the ultrasonic imaging apparatus is a determining entity, it may transmit a command signal to switch into the low-power mode to the wireless probe based on the determination result, thereby leading the wireless probe to be switched into the low-power mode. In another example, if the wireless probe is the determining entity, it may be switched into the low-power mode by itself, based on the determination result.

Otherwise, if the condition is better than the predetermined condition in operation 1330, the ultrasonic imaging system may determine that the wireless probe is able to normally capture ultrasound images and provide them to the user, thereby remaining in the scanning mode. The predetermined condition may be set for each of various parameters as described above, and stored in the memory unit of the ultrasonic imaging apparatus or wireless probe.

The ultrasonic imaging system may figure out position information between the wireless probe and the ultrasonic imaging apparatus, and determine whether an ultrasound image is currently being acquired, based on the position information, in operation 1340. In other words, in light of the position information between the wireless probe and the ultrasonic imaging apparatus, e.g., the position information indicating that the wireless probe and the ultrasonic imaging apparatus are far away from each other, if it is determined that diagnosis of an object is not being currently made by acquiring an ultrasound image, the ultrasonic imaging system may determine to switch the wireless probe into the low-power mode, in operation 1320.

Furthermore, the ultrasonic imaging system may determine whether the ultrasound image includes an object, and determine whether to switch into the low-power mode based on the determination result of whether the ultrasound image includes an object, in operation 1350. For example, data about air images for the respective modes may be stored in the memory unit of the ultrasonic imaging apparatus or the wireless probe. Accordingly, the ultrasonic imaging system may determine whether it is in the scanning mode by comparing the data stored in the memory unit with an ultrasound image acquired in real time.

For example, if it is determined that an object is currently being scanned due to the significant difference between the air image and the ultrasound image, the ultrasonic imaging system may remain in the scanning mode of the wireless probe, in operation 1370. Otherwise, if it is determined that an object is not currently being scanned due to close similarity to the air image, the ultrasonic imaging system may calculate non-scanning time, i.e., calculate whether the waiting time has passed, in operation 1360. The waiting time refers to a period of time for acquiring or capturing an ultrasound image determined as the air image. Accordingly, if the waiting time has passed, the ultrasonic imaging system may determine to switch the wireless probe into the low-power mode, in operation 1320.

As described above, the ultrasonic imaging system may collect various information to determine whether to switch into the low-power mode. Determination of whether to switch into the low-power mode is not limited to what are described in connection with the drawings, and may be made by combining a condition of the wireless communication network, position information, a result determined from image information, and whether the waiting time has passed and determining based on the combination whether it is difficult to normally provide the ultrasound image or that ultrasound image scanning is not underway.

As described above, at least one of the ultrasonic imaging apparatus and the wireless probe, which constitute the ultrasonic imaging system, may determine whether to switch into the low-power mode. Operations performed by each of the wireless probe and ultrasonic imaging apparatus will now be described.

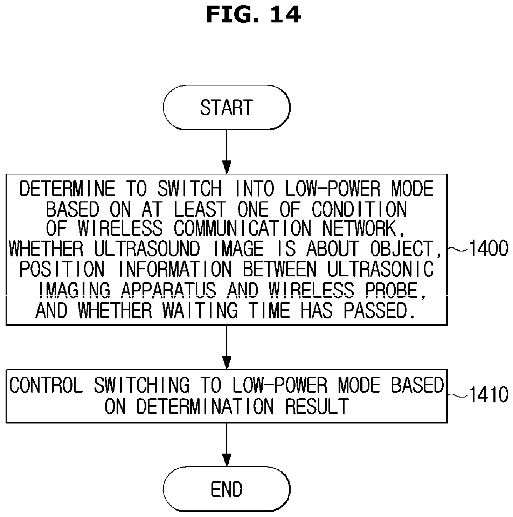

FIG. 14 is a flowchart illustrating operation of a wireless probe, according to an embodiment of the present disclosure.

In an embodiment, a wireless probe may be switched into the low-power mode if it is determined that the user is not actually performing scanning even while in the scanning mode. Whether to switch into the low-power mode may be determined based on various criteria.

For example, whether to switch the wireless probe into the low-power mode may be determined based on various operating status of the wireless probe. In an embodiment, the wireless probe may determine whether to switch into the low-power mode based on at least one of a condition of a wireless communication network, whether an ultrasound image includes an object, position information between the ultrasonic imaging apparatus and the wireless probe, and whether the waiting time has passed, in operation 1400.

The condition of a wireless communication network refers to connection status of the wireless communication network that connects the wireless probe and the ultrasonic imaging apparatus, which may be determined based on various parameters that may be set as references to determine the condition of the wireless communication network.

Whether the ultrasound image is about an object may be determined by obtaining image information from the ultrasound image through image processing and determining whether the ultrasound image includes an object based on the obtained image information.

The position information may be figured out by deriving distance information and directional information based on receive signal strength and radiation pattern information. The wireless probe may include three communication modules at different positions. Accordingly, the position information may be figured out by triangulation.

Alternatively, with position measurement sensors built into the wireless probe and the ultrasonic imaging apparatus, the wireless probe may receive position information of the ultrasonic imaging apparatus from the ultrasonic imaging apparatus over a wireless communication network and thus determine position information between both devices.

The waiting time is set in advance in the wireless probe, and if the waiting time has passed, the wireless probe may determine that the user has no intent to perform ultrasound image scanning, thereby determining to switch into the low-power mode.

If at least N cases are met, where N ≥ 1, among cases that a condition of the wireless communication network is unable to normally deliver an ultrasound image or echo ultrasound signal, that the ultrasound image contains no object, that it is not determined that normal ultrasound image scanning is underway in light of the position information between the ultrasonic imaging apparatus and the wireless probe, that the waiting time has passed, the wireless probe may be switched into the low-power mode. Accordingly, if the user is not actually performing scanning even while in the scanning mode, the wireless probe may be switched into the low-power mode, thereby preventing unnecessary power consumption.

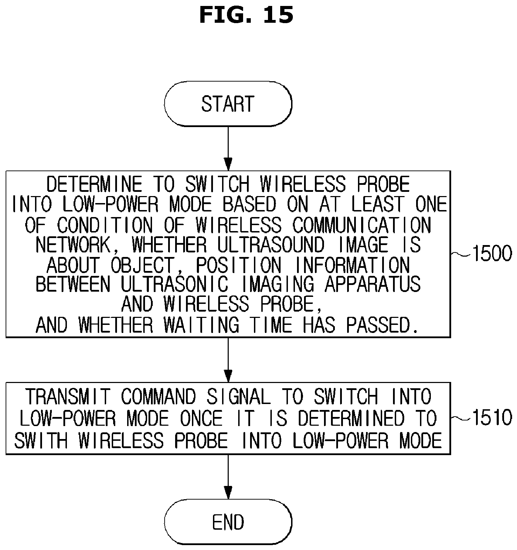

FIG. 15 is a flowchart illustrating operation of an ultrasonic imaging apparatus, according to an embodiment of the present disclosure.

An ultrasonic imaging apparatus may determine whether to switch the wireless probe into the low-power mode by determining status of operation of the wireless probe. If the user is not actually performing scanning or the ultrasound image may not be provided to the user in real time even while the wireless probe is in the normal power mode, such as normal scanning mode, the wireless probe may be switched into the low-power mode.

For example, the ultrasonic imaging apparatus may determine whether to switch the wireless probe into the low-power mode by receiving status of operation of the wireless probe over the wireless communication network or determining connection status of the wireless communication network.

In an embodiment, the ultrasonic imaging apparatus may determine whether to switch the wireless probe into the low-power mode based on at least one of a condition of the wireless communication network, whether the ultrasound image contains an object, position information between the ultrasonic imaging apparatus and the wireless probe, and whether the waiting time has passed, in operation 1500. This will not be described any further because it was already described above.

Once it is determined to switch into the low-power mode, the ultrasonic imaging apparatus may transmit a switching command signal to request the wireless probe to be switched into the low-power mode over the wireless communication network, in operation 1510. In response to reception of the switching command signal, the wireless probe may be switched into the low-power mode, thereby preventing unnecessary power consumption.

According to embodiments of the present disclosure of a wireless probe and ultrasonic imaging apparatus, whether to switch into a low-power mode may be more accurately determined, thereby enabling more efficient power management.

The method according to the embodiments of the present disclosure may be implemented in program instructions which are executable by various computing means and recorded in computer-readable media. The computer-readable media may include program instructions, data files, data structures, etc., separately or in combination. The program instructions recorded on the computer-readable media may be designed and configured specially for the present disclosure, or may be well-known to people having ordinary skill in the art of computer software. Examples of the computer readable recording medium include read-only memories (ROMs), random-access memories (RAMs), Compact Disc (CD)-ROMs, magnetic tapes, floppy disks, optical data storage devices, etc. The computer readable recording medium can also be distributed over network-coupled computer systems so that the computer readable code is stored and executed in a distributed fashion. Examples of the program instructions include not only machine language codes but also high-level language codes which are executable by various computing means using an interpreter. The aforementioned hardware devices may be configured to operate as one or more software modules to carry out exemplary embodiments of the present disclosure, and vice versa.

Although the present disclosure is described with reference to some embodiments as described above and accompanying drawings, it will be apparent to those ordinary skilled in the art that various modifications and changes can be made to the embodiments, as far as they fall within the scope of the appended claims. For example, the aforementioned method may be performed in different order, and/or the aforementioned systems, structures, devices, circuits, etc., may be combined in different combinations from what is described above, and/or replaced or substituted by other components or equivalents thereof, to obtain appropriate results.

Therefore, other implementations, other embodiments, and equivalents thereof may fall within the following claims.

## Claims

1. An ultrasonic wireless probe (100) comprising
a communication unit (140) configured to exchange various data with the wireless ultrasonic probe over a wireless communication network;
a determiner (170) configured to obtain distance information and directional information between the wireless probe (100) and an ultrasonic imaging apparatus (200) calculated using triangulation or radiation pattern information of the wireless communication network, and to determine whether to switch the wireless probe (100) into a low-power mode based on the distance information and the directional information between the wireless probe (100) and the ultrasonic imaging apparatus (200); and
a probe controller (180) for controlling switching into the low-power mode based on the determined distance information and the determined directional information when the wireless probe is located opposite to the frontal direction of a display of the ultrasonic imaging apparatus.

2. The wireless probe of claim 1, wherein the determiner (170) is configured to determine whether to switch into a low-power mode based on distance information and directional information between the wireless probe (100) and the ultrasonic imaging apparatus (200), wherein the distance information is calculated based on a receive signal strength.

3. The wireless probe (100) of claim 1, wherein the determiner is configured to determine a condition of the wireless communication network based on at least one of transfer rate, signal quality, signal to noise ratio (SNR), and receive signal strength indication (RSSI) of a wireless signal over the wireless communication network.

4. The wireless probe (100) of claim 3, wherein the determiner (260) is configured to determine the condition of the wireless communication network based on a signal threshold received by a user or set in advance.

5. The wireless probe (100) of any of the preceding claims, wherein the determiner (170) is configured to determine whether the ultrasound image corresponds to an air image using image information obtained through image processing on the ultrasound image.

6. The wireless probe (100) of any of the preceding claims, wherein the determiner (170) is configured to further determine whether to switch into a low-power mode based on whether the distance information between the wireless probe (100) and the ultrasonic imaging apparatus (200) is within a predetermined distance, the distance information being calculated based on receive signal strength.

7. An ultrasonic imaging apparatus (200) comprising:
a display (220) configured to display an ultrasound image;
a communication unit (230) configured to exchange various data with a wireless ultrasonic probe (100) over a wireless communication network;
a determiner (260) configured to determine whether to switch the wireless ultrasonic probe (100) into a low-power mode based on distance information and directional information between the wireless probe (100) and the ultrasonic imaging apparatus (200) calculated using triangulation or radiation pattern information of the wireless communication network; and
a main controller to control a communication unit to transmit a command signal to the wireless probe (100) to be switched into the low-power mode, once it is determined to switch the wireless probe (100) into the low-power mode.

8. The ultrasonic imaging apparatus (200) of claim 7, wherein the determiner (260) is configured to determine a condition of the wireless communication network based on at least one of transfer rate, signal quality, signal to noise ratio (SNR), and receive signal strength indication (RSSI) of a wireless signal over the wireless communication network.

9. The ultrasonic imaging apparatus (200) of claim 8, wherein the determiner (260) is configured to determine the condition of the wireless communication network based on a signal threshold received by a user or set in advance.

10. The ultrasonic imaging apparatus (200) of claim 7, 8 or 9, wherein the determiner (260) is configured to determine whether the ultrasound image corresponds to an air image using image information obtained through image processing on the ultrasound image.

11. The ultrasonic imaging apparatus (200) of any of the claims 7-10, wherein the determiner (260) is further configured to determine whether to switch into a low-power mode based on whether the distance information between the wireless probe (100) and the ultrasonic imaging apparatus (200) is within a predetermined distance, the distance information being calculated based on receive signal strength.

12. An ultrasonic imaging system comprising an ultrasonic imaging apparatus (200) according to any of claims 7-11, and an wireless probe (100) is the wireless probe (100) as defined in any of the claims 1-6.

## Patentansprüche

1. Drahtlose Ultraschallsonde (100) mit:
einer Kommunikationseinheit (140), die dazu ausgelegt ist, verschiedene Daten mit der drahtlosen Ultraschallsonde innerhalb einem drahtlosen Kommunikationsnetzwerk auszutauschen;
einem Bestimmer (170), der dazu ausgelegt ist, Abstandsinformationen und Richtungsinformationen zwischen der drahtlosen Sonde (100) und einer Ultraschallbildgebungsvorrichtung (200) zu erhalten, die mittels Triangulations- oder Strahlungsmusterinformationen des drahtlosen Kommunikationsnetzwerks berechnet werden, und zu bestimmen, ob die drahtlose Sonde (100) in einen Niedrigleistungsmodus auf der Grundlage der Abstandsinformationen und Richtungsinformationen zwischen der drahtlosen Sonde (100) und der Ultraschallbildgebungsvorrichtung (200) umgeschaltet werden soll;
einer Sondensteuerung (180) zum Steuern des Umschaltens in den Niedrigleistungsmodus auf der Grundlage der bestimmten Abstandsinformationen und den bestimmten Richtungsinformationen, wenn die drahtlose Sonde entgegengesetzt der Frontrichtung einer Anzeige der Ultraschallbildgebungsvorrichtung ausgerichtet ist.

2. Drahtlose Sonde nach Anspruch 1, wobei der Bestimmer (170) ausgelegt ist, zu bestimmen, ob in einen Niedrigleistungsmodus umgeschaltet werden soll, und zwar auf der Grundlage der Abstandsinformationen und Richtungsinformationen zwischen der drahtlosen Sonde (100) und der Ultraschallbildgebungsvorrichtung (200), wobei die Abstandsinformationen auf der Grundlage der Stärke eines empfangenen Signals berechnet werden.

3. Drahtlose Sonde (100) nach Anspruch 1, wobei der Bestimmer dazu ausgelegt ist, einen Zustand des drahtlosen Kommunikationsnetzwerks zu bestimmen, und zwar auf der Grundlage der Übertragungsrate, der Signalqualität, des Signal-Rausch-Verhältnisses (SNR, Signal to Noise Ratio) und/oder einer RSSI (Received Signal Strength Indication, Anzeige der Empfangssignalstärke) eines drahtlosen Signals innerhalb des drahtlosen Kommunikationsnetzwerks.

4. Drahtlose Sonde (100) nach Anspruch 3, wobei der Bestimmer (260) dazu ausgelegt ist, den Zustand des drahtlosen Kommunikationsnetzwerks auf der Grundlage eines von einem Benutzer empfangenen oder im Voraus eingestellten Signalschwellenwerts zu bestimmen.

5. Drahtlose Sonde (100) nach einem der vorhergehenden Ansprüche, wobei der Bestimmer (170) ausgelegt ist, zu bestimmen, ob das Ultraschallbild einem Luftbild entspricht, und zwar mittels Bildinformationen, die durch Bildverarbeitung des Ultraschallbildes erhalten werden.

6. Drahtlose Sonde (100) nach einem der vorhergehenden Ansprüche, wobei der Bestimmer (170) ausgelegt ist, weiterhin zu bestimmen, ob in einen Niedrigleistungsmodus umgeschaltet werden soll, und zwar auf der Grundlage dessen, ob die Abstandsinformationen zwischen der drahtlosen Sonde (100) und der Ultraschallbildgebungsvorrichtung (200) innerhalb eines vorbestimmten Abstands sind, wobei die Abstandsinformationen auf der Grundlage der Stärke eines empfangenen Signals berechnet werden.

7. Ultraschallbildgebungsvorrichtung (200) mit:
einer Anzeige (220), die dazu ausgelegt ist, ein Ultraschallbild anzuzeigen;
einer Kommunikationseinheit (230), die dazu ausgelegt ist, verschiedene Daten mit einer drahtlosen Ultraschallsonde (100) innerhalb einem drahtlosen Kommunikationsnetzwerk auszutauschen;
einem Bestimmer (260), der ausgelegt ist, zu bestimmen, ob die drahtlose Sonde (100) in einen Niedrigleistungsmodus auf der Grundlage von Abstandsinformationen und Richtungsinformationen zwischen der drahtlosen Sonde (100) und einer Ultraschallbildgebungsvorrichtung (200) umgeschaltet werden soll, die mittels Triangulations- oder Strahlungsmusterinformationen des drahtlosen Kommunikationsnetzwerks berechnet werden; und
einer Hauptsteuerung zum Steuern einer Kommunikationseinheit, damit sie ein Steuersignal an die drahtlose Sonde (100) sendet, so dass diese in den Niedrigleistungsmodus umgeschaltet wird, wenn es bestimmt wird, dass die drahtlose Sonde (100) in den Niedrigleistungsmodus umgeschaltet werden soll.

8. Ultraschallbildgebungsvorrichtung (200) nach Anspruch 7, wobei der Bestimmer (260) dazu ausgelegt ist, einen Zustand des drahtlosen Kommunikationsnetzwerks zu bestimmen, und zwar auf der Grundlage der Übertragungsrate, der Signalqualität, des Signal-Rausch-Verhältnisses (SNR, Signal to Noise Ratio) und/oder einer RSSI (Received Signal Strength Indication, Anzeige der Empfangssignalstärke) eines drahtlosen Signals innerhalb des drahtlosen Kommunikationsnetzwerks.

9. Ultraschallbildgebungsvorrichtung nach Anspruch 8, wobei der Bestimmer (260) dazu ausgelegt ist, den Zustand des drahtlosen Kommunikationsnetzwerks auf der Grundlage eines von einem Benutzer empfangenen oder im Voraus eingestellten Signalschwellenwerts zu bestimmen.

10. Ultraschallbildgebungsvorrichtung (200) nach Anspruch 7, 8 oder 9, wobei der Bestimmer (260) ausgelegt ist, zu bestimmen, ob das Ultraschallbild einem Luftbild entspricht, und zwar mittels Bildinformationen, die durch Bildverarbeitung des Ultraschallbildes erhalten werden.

11. Ultraschallbildgebungsvorrichtung (200) nach einem der Ansprüche 7 bis 10, wobei der Bestimmer (260) weiterhin ausgelegt ist, zu bestimmen, ob in einen Niedrigleistungsmodus umgeschaltet werden soll, auf der Grundlage dessen, ob die Abstandsinformationen zwischen der drahtlosen Sonde (100) und der Ultraschallbildgebungsvorrichtung (200) innerhalb eines vorbestimmten Abstands sind, wobei die Abstandsinformationen auf der Grundlage der Stärke eines empfangenen Signals berechnet werden.

12. Ultraschallbildgebungssystem mit einer Ultraschallbildgebungsvorrichtung (200) nach einem der Ansprüche 7 bis 11 und einer drahtlosen Sonde (100), die die drahtlose Sonde (100) nach einem der Ansprüche 1 bis 6 ist.

## Revendications

1. Sonde échographique sans fil (100) comprenant :
une unité de communication (140) conçue pour échanger diverses données avec la sonde sans fil échographique par le biais d'un réseau de communication sans fil ;
un déterminateur (170) conçu pour obtenir des informations de distance et des informations de direction entre la sonde sans fil (100) et un dispositif d'imagerie échographique (200) calculées à l'aide d'informations de triangulation ou de diagramme de rayonnement du réseau de communication sans fil, et pour déterminer s'il y a lieu de faire passer la sonde sans fil (100) en un mode basse puissance compte tenu des informations de distance et des informations de direction entre la sonde sans fil (100) et le dispositif d'imagerie échographique (200) ;
un contrôleur de sonde (180) destiné à contrôler le passage en mode basse puissance compte tenu des informations de distance déterminées et des informations de direction déterminées lorsque la sonde sans fil est située à l'opposé d'un sens avant d'un écran du dispositif d'imagerie échographique.

2. Sonde sans fil selon la revendication 1, dans laquelle le déterminateur (170) est conçu pour déterminer s'il y a lieu d'effectuer le passage en mode basse puissance compte tenu des informations de distance et des informations de direction entre la sonde sans fil (100) et le dispositif d'imagerie échographique (200), lesdites informations de distance étant calculées compte tenu de l'intensité d'un signal reçu.

3. Sonde sans fil (100) selon la revendication 1, dans laquelle le déterminateur est conçu pour déterminer un état du réseau de communication sans fil compte tenu du débit, de la qualité, du rapport signal sur bruit (RSB) et/ou d'un indicateur de l'intensité du signal reçu (RSSI, received signal strength indication) propres à un signal sans fil sur le réseau de communication sans fil.

4. Sonde sans fil (100) selon la revendication 3, dans laquelle le déterminateur (260) est conçu pour déterminer l'état du réseau de communication sans fil compte tenu d'un seuil de signal reçu par un utilisateur ou défini par avance.

5. Sonde sans fil (100) selon l'une quelconque des revendications précédentes, dans laquelle le déterminateur (170) est conçu pour déterminer si l'image échographique correspond à une image due à l'air à l'aide d'informations d'image obtenues par un traitement d'image réalisé sur l'image échographique.

6. Sonde sans fil (100) selon l'une quelconque des revendications précédentes, dans laquelle le déterminateur (170) est conçu pour déterminer en outre s'il y a lieu de passer en mode basse puissance compte tenu du fait que les informations de distance entre la sonde sans fil (100) et le dispositif d'imagerie échographique (200) sont à moins d'une distance prédéterminée ou non, les informations de distance étant calculées compte tenu de l'intensité d'un signal reçu.

7. Dispositif d'imagerie échographique (200) comprenant :
un écran (220) conçu pour afficher une image échographique,
une unité de communication (230) conçue pour échanger diverses données avec une sonde sans fil échographique (100) par le biais d'un réseau de communication sans fil,
un déterminateur (260) conçu pour déterminer s'il y a lieu de faire passer la sonde sans fil (100) en un mode basse puissance compte tenu d'informations de distance et d'informations de direction entre la sonde sans fil (100) et un dispositif d'imagerie échographique (200) calculées à l'aide d'informations de triangulation ou de diagramme de rayonnement du réseau de communication sans fil, et
un contrôleur principal destiné à contrôler une unité de communication de façon qu'elle émette un signal de commande à destination de la sonde sans fil (100) de manière qu'elle passe en mode basse puissance, une fois qu'il a été déterminé de faire passer la sonde sans fil (100) en mode basse puissance.

8. Dispositif d'imagerie échographique (200) selon la revendication 7, dans lequel le déterminateur (260) est conçu pour déterminer un état du réseau de communication sans fil compte tenu du débit, de la qualité, du rapport signal sur bruit (RSB) et/ou d'un indicateur de l'intensité du signal reçu (RSSI, received signal strength indication) propres à un signal sans fil sur le réseau de communication sans fil.

9. Dispositif d'imagerie échographique selon la revendication 8, dans lequel le déterminateur (260) est conçu pour déterminer l'état du réseau de communication sans fil compte tenu d'un seuil de signal reçu par un utilisateur ou défini par avance.

10. Dispositif d'imagerie échographique (200) selon la revendication 7, 8 ou 9, dans lequel le déterminateur (260) est conçu pour déterminer si l'image échographique correspond à une image due à l'air à l'aide d'informations d'image obtenues par un traitement d'image réalisé sur l'image échographique.

11. Dispositif d'imagerie échographique (200) selon l'une quelconque des revendications 7 à 10, dans lequel le déterminateur (260) est conçu en outre pour déterminer s'il y a lieu d'effectuer un passage en mode basse puissance compte tenu du fait que les informations de distance entre la sonde sans fil (100) et le dispositif d'imagerie échographique (200) sont à moins d'une distance prédéterminée ou non, les informations de distance étant calculées compte tenu de l'intensité d'un signal reçu.

12. Système d'imagerie échographique comprenant un dispositif d'imagerie échographique (200) selon l'une quelconque des revendications 7 à 11, et une sonde sans fil (100) consistant en la sonde sans fil (100) selon l'une quelconque des revendications 1 à 6.
